# EUROPEAN PATENT APPLICATION

(11) **EP 4 509 508 A1**
(43) Date of publication of application: **19.02.2025**
(21) Application number: 23787839.2
(22) Date of filing: 14.04.2023
(51) Int. Cl.: C07D 471/04, A61P 25/28, A61P 25/00, A61P 17/14, A61P 25/08, A61P 35/02, A61P 35/00, A61P 27/02, A61K 31/444

(54) **FREE-STATE PLX5622 CRYSTAL FORM AND PREPARATION METHOD THEREFOR**

(30) Priority: 15.04.2022 CN 202210400114
(71) Applicant: SHANGHAI HAOYUAN MEDCHEMEXPRESS CO., LTD., Shanghai 201210 (CN)
(72) Inventor: CAO, Ming, Shanghai 201210 (CN); WANG, Guangyong, Shanghai 201210 (CN); HUANG, Zongsui, Shanghai 201210 (CN); CHEN, Yufeng, Shanghai 201210 (CN); ZHANG, Feixiong, Shanghai 201210 (CN); YANG, Shunping, Shanghai 201210 (CN); LI, Chaoping, Shanghai 201210 (CN); JIAO, Lei, Shanghai 201210 (CN); ZHOU, Zhiguo, Shanghai 201210 (CN); YANG, Shaobo, Shanghai 201210 (CN); GAO, Qiang, Shanghai 201210 (CN); ZHENG, Baofu, Shanghai 201210 (CN)
(74) Representative: dompatent von Kreisler Selting Werner - Partnerschaft von Patent- und Rechtsanwälten mbB
(86) International application number: PCT/CN2023/088431
(87) International publication number: WO 2023/198197

(57) **Abstract**

The present invention provides a free-state PLX5622 crystal form and a preparation method therefor, and in particular, provides crystal forms A, B, C, D, E and F of a compound of formula I which is a free alkali PLX5622 with the chemical name of 6-fluoro-N-((5-fluoro-2-methoxypyridin-3-yl)methyl)-5-((5-methyl-1H-pyrrolo[2,3-b]pyridin-3-yl)methyl) pyridin-2-amine, and preparation methods therefor. The structural formula of the compound of formula I is as follows. According to the present invention, the crystal form A has a relatively stable physicochemical property; the crystal form B has a better effect of eliminating microglial cells and a more stable drug effect; the hydrate crystal form C, the solvate crystal form D, the crystal form E and the crystal form F have better crystallinity, granularity and fluidity from the solid form, and the preparation methods for the crystal forms are simple and convenient, thereby providing more choices for the medicinal crystal form research of the compound of formula I which is the free alkali PLX5622.

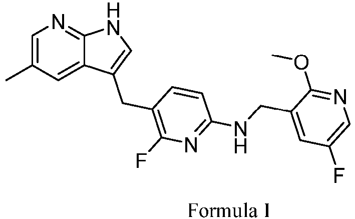

## Description

The present application claims the right of priority of the Chinese patent application No. 202210400114.X, filed on Apr. 15th, 2022, which is hereby incorporated by reference in its entirety.

### TECHNICAL FIELD

The invention relates to a novel crystal form of free-state PLX5622 and preparation method thereof, belonging to the field of pharmaceuticals and chemical technology.

### BACKGROUND

PLX5622 is a CSFIR colony-stimulating factor-1 receptor inhibitor, and its chemical structure is shown in Formula I:

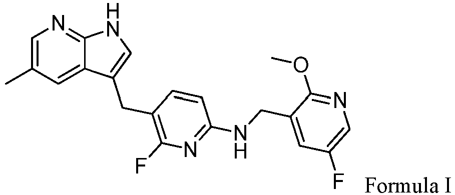

PLX5622 is a highly selective, blood-brain barrier permeable, orally effective CSFIR inhibitor (IC₅₀ = 0.016 µM; Kᵢ =5.9 nM). PLX5622 can deplete microglia, reduce number of perivascular macrophages and circulating immune cells. Studies found that eliminating microglia with the CSFIR inhibitor PLX5622 can partially prevent short-term memory impairment caused by long-term infusion of Ang II, and the improvement in cognition by PLX5622 is unrelated to changes in blood-brain barrier permeability. This represents an important step in the development of CSF1R therapys, and further research is needed to develop a treatment regimen to achieve rapid and safe depletion of microglia in patients in a short period of time.

PLX5622 is prepared in accordance with scheme 25 of CN102695417A(WO2011057022A1), wherein the PLX5622 compound obtained from the reaction is concentrated and subjected to purification using a silica gel column (eluted with a hexane solution of 20-100% ethyl acetate), followed by concentration to yield an amorphous white solid. The free base of PLX5622 was disclosed in Nature Communications, 2019, 10(1): 3758, with a melting point of 189°C.

However, up to the present time, no crystal form of PLX5622 has been discovered. During the in-depth pharmaceutical research of PLX5622, the inventor unexpectedly discovered several different crystal forms. Any aspect of the specific crystal forms A to F of compound I described in the invention remains undisclosed, and the crystal forms of compound I provided by the invention has outstanding medicinal efficacy in microglia cell clearance.

### DESCRIPTION OF THE INVENTION

According to the aforementioned technical background, the invention satisfies these needs by providing solid form of the compound of formula I. The invention provides crystal forms of compound of formula I.

The invention also provides a pharmaceutical composition comprising solid forms of compound of formula I. The invention also provides method for preparing solid forms and their use in the preparation of drugs for treating CSF1R-mediated related diseases.

The invention provides polymorphic crystal forms of compound I, and preferred embodiments offering crystal forms A, B, C, D, E and F of free-state PLX5622 (compound I) with excellent stability and their preparation methods.

The free-state PLX5622(compound of formula I) described in the invention, with chemical name: 6-Fluoro-N-((5 -fluoro-2-methoxypyridin-3 -yl)methyl)-5 -((5 -methyl-1H-pyrrolo[2, 3-b]pyridin-3-yl)methyl)pyridin-2-amine, has a structural formula I as shown:

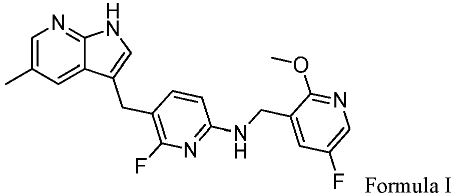

The object of the present invention is to provide a crystal form A of compound of formula I, and one embodiment relates to a free-state PLX5622 (compound of formula I) crystal form A. The crystal form A of compound of formula I has an X-ray powder diffraction pattern comprising characteristic absorption peaks at diffraction angle 2Θ values of 8.60±0.2°, 10.50±0.2°, and 13.80±0.2°using Cu-Ku radiation.

The invention also provides a crystal form A of compound of formula I, wherein the form A is an anhydrous form.

In some embodiments, a crystal form A of compound I has no solvent, and the solvent comprising water.

The invention provides a crystal form A of compound of formula I, which has an X-ray powder diffraction pattern comprising absorption characteristic peaks at diffraction angle 2θ values of 8.60±0.2°, 13.80±0.2°, 18.38±0.2°, and 20.90±0.2°.

The invention provides a crystal form A of compound of formula I, which has an X-ray powder diffraction pattern comprising characteristic absorption peaks at diffraction angle 2Θ values of 8.60±0.2°, 10.50±0.2°, 13.80±0.2°, 14.66±0.2°, 17.18±0.2°, 18.38±0.2°, 20.90±0.2° and 25.98±0.2°.

The invention provides a crystal form A of compound of formula I, which has an X-ray powder diffraction pattern comprising characteristic absorption peaks at diffraction angle 2Θ values of 8.60±0.2°, 9.18±0.2°, 10.50±0.2°, 12.94±0.2°, 13.80±0.2°, 14.66±0.2°, 16.67±0.2°, 17.18±0.2°, 17.70±0.2°, 18.38±0.2°, 18.90±0.2°, 19.46±0.2°, 20.08±0.2°, 20.90±0.2°, 22.02±0.2°, 23.86±0.2°, 24.74±0.2°, 25.62±0.2°, 25.98±0.2°, 26.70±0.2°, 27.40±0.2°, 27.70±0.2°, 28.48±0.2°, 29.44±0.2°, 29.76±0.2°, 30.86±0.2° and 31.88±0.2°.

In one embodiment, the invention provides a method for preparing a crystal form A of compound of formula I, wherein the method comprises recrystallizing the crystal form of the compound I from mixture of alcoholic solvents such as methanol, ethanol, isopropanol and/or water.

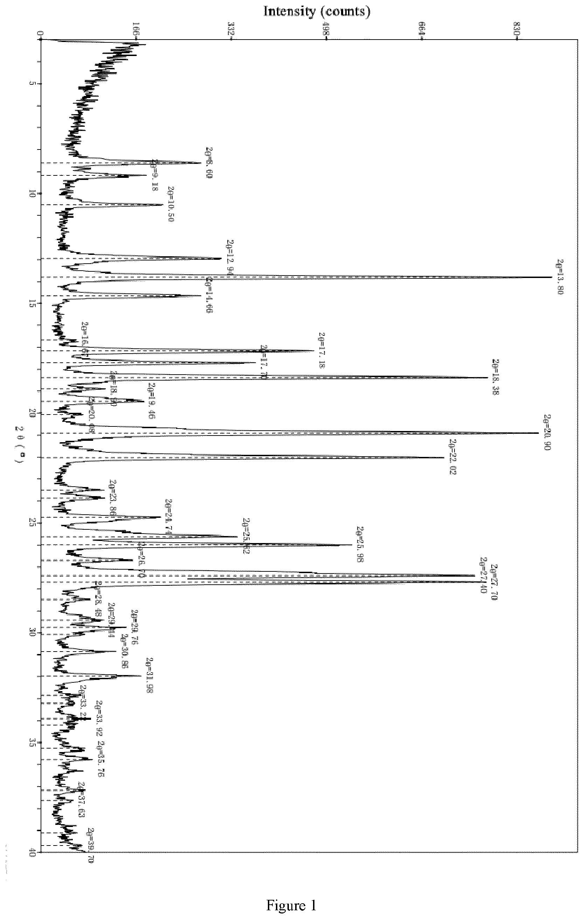

Further preferably, the crystal form A of compound of formula I has an X-ray powder diffraction pattern substantially as shown in Figure 1.

Another embodiment relates to a free-state PLX5622 (compound of formula I) crystal form B. The crystal form B of compound of formula I has an X-ray powder diffraction pattern comprising characteristic absorption peaks at diffraction angle 2Θ values of 5.9±0.2°, 15.6±0.2°, and 17.2±0.2°using Cu-Ku radiation.

In some embodiments, a crystal form B of compound of formula I has no solvent, and the solvent comprising water.

Another object of the invention is to provide a crystal form B of compound of formula I, wherein the form B is an anhydrous form.

Preferably, the X-ray powder diffraction pattern of crystal form B of compound of formula I of the invention further has characteristic peaks at one or more of the following places with 2Θ values: 9.18±0.2°, 17.84±0.2°, 21.64±0.2°, 22.50±0.2°, and 27.64±0.2°; more preferably, the X-ray powder diffraction pattern further has characteristic peaks at one or more of the following places with 2Θ values: 11.70±0.2°, 14.16±0.2°, 19.86±0.2°, 21.99±0.2°, 23.88±0.2°, 24.28±0.2°, 24.52±0.2°, 28.10±0.2° and 28.40±0.2°.

The invention provides a crystal form B of compound of formula I, which has an X-ray powder diffraction pattern comprising characteristic absorption peaks at diffraction angle 2θ values of 5.90±0.2°, 15.65±0.2°, 17.20±0.2° and 22.50±0.2°.

The invention provides a crystal form B of compound of formula I, which has an X-ray powder diffraction pattern comprising characteristic absorption peaks at diffraction angle 2Θ values of 5.90±0.2°, 9.18±0.2°, 15.65±0.2°, 17.20±0.2°, 17.84±0.2°, 21.64±0.2°, 22.50±0.2° and 27.64±0.2°.

The invention provides a crystal form B of compound of formula I, which has an X-ray powder diffraction pattern comprising characteristic absorption peaks at diffraction angle 2Θ values of 5.90±0.2°, 9.18±0.2°, 11.70±0.2°, 14.16±0.2°, 15.65±0.2°, 17.20±0.2°, 17.84±0.2°, 19.86±0.2°, 21.64±0.2°, 21.99±0.2°, 22.50±0.2°, 23.88±0.2°, 24.28±0.2°, 24.52±0.2°, 27.64±0.2°, 28.10±0.2° and 28.40±0.2°.

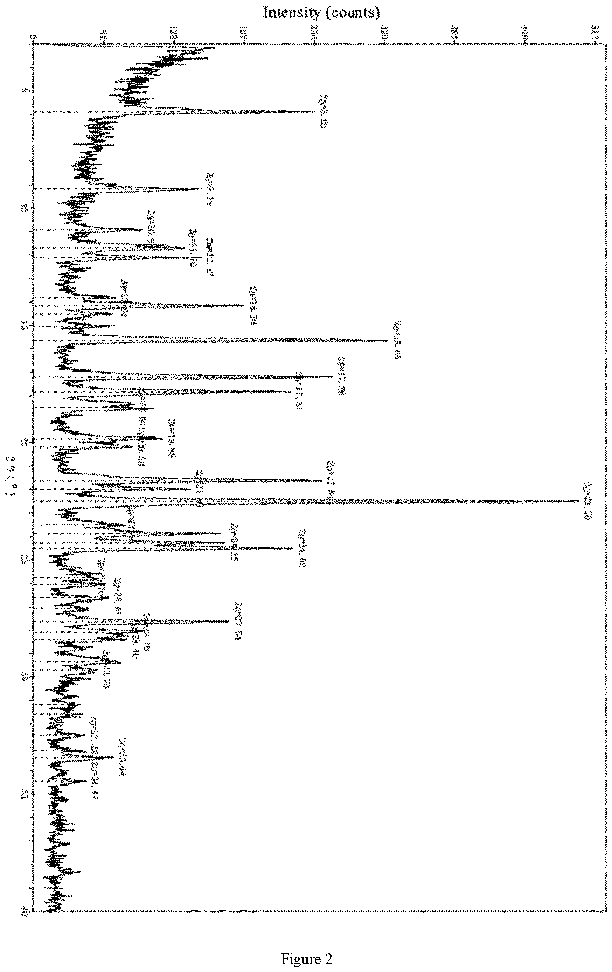

Further preferably, the crystal form B of compound of formula I has an XRPD pattern as shown in Figure 2.

Further preferably, the crystal form B of compound of formula I has a differential scanning calorimetry (DSC) curve with two endothermic peaks at 181°C ± 4°C and 188°C ± 4°C.

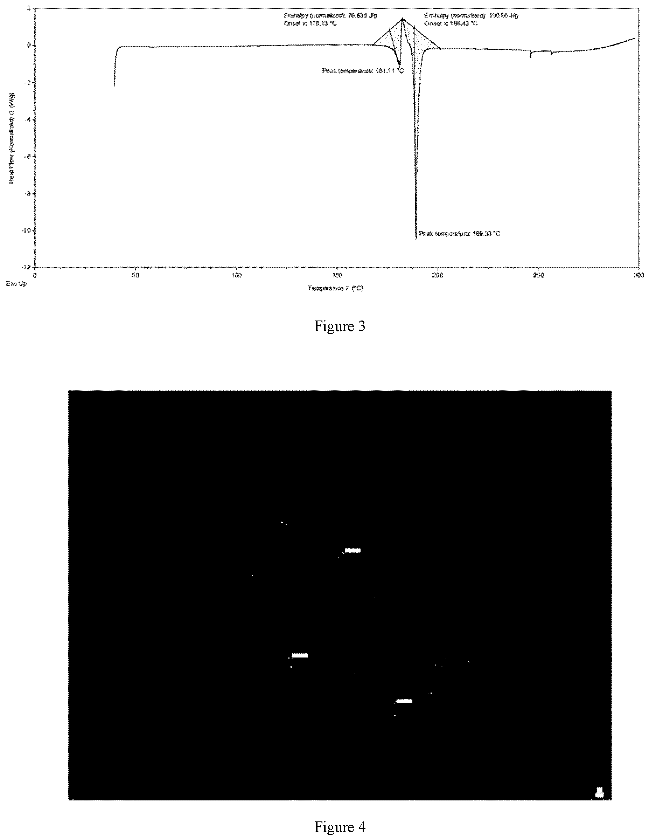

Further preferably, the crystal form B of compound of formula I has a differential scanning calorimetry curve as shown in Figure 3.

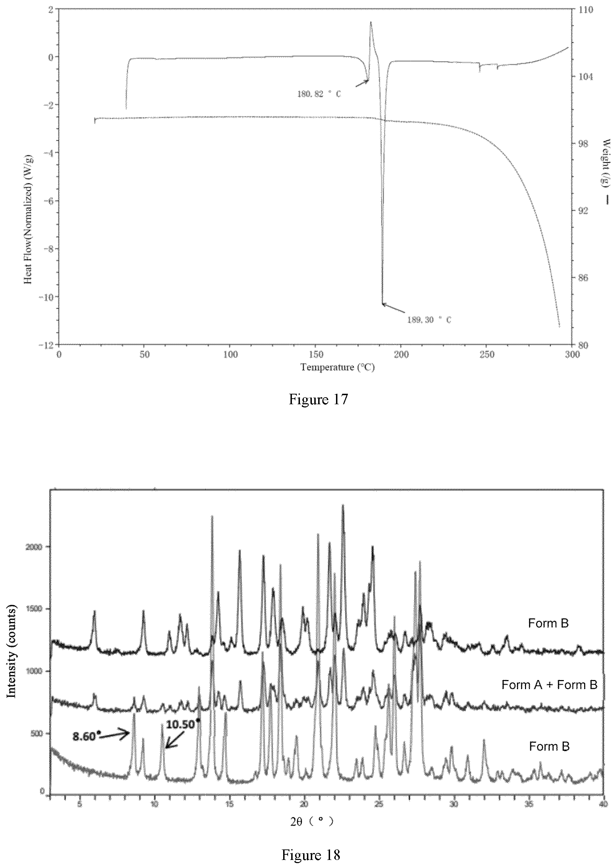

Further preferably, the crystal form B of compound of formula I has a thermal gravimetric analysis (TGA) curve substantially as shown in Figure 17.

Further preferably, the crystal form B of compound of formula I has a DSC curve as shown in Figure 3, wherein the form B at least has two endothermic peaks at 181°C ± 4°C and 188°C ± 4 °C.

Further preferably, the DSC analysis method of crystal form B of compound of formula I as disclosed in the invention is as follows: the scanning rate is 10°C/min.

The invention also provides a method for preparing a crystal form B of compound of formula I by rapidly removing the solvent.

The method for preparing the crystal form B of compound of formula I comprises dissolving compound of formula I in a solvent of ethers, alkanes, alcohols, esters, or any combination thereof, then removing the solvent to obtain the compound of formula I. The method for preparing a crystal form B of compound of formula I comprises the step of removing the solvent rapidly, such as obtaining product by distillation or spray drying.

The method for preparing a crystal form B of compound of formula I further comprises the step of removing the solvent rapidly or such as dissolving in an ether solvent and then spray drying. The volume-to-mass ratio of the ether solvent to compound of formula I can be 15-100 mL/g, for example, 30 mL/g, wherein the ether solvent can be tetrahydrofuran.

In a preferred embodiment of the invention, a method for preparing a crystal form B of compound of formula I is provided, comprising the following steps:
(1) dissolving compound of formula I in a tetrahydrofuran solution;
(2) aapidly removing the solvent by spray drying;
(3) obtaining the crystal form B of compound of formula I;
(4) further drying the crystal form B of compound of formula I obtained by step (3).

The invention also provides a method for preparing a crystal form B of compound of formula I using a crystallization method.

The method for preparing a crystal form B of compound of formula I comprises dissolving compound of formula I in a solvent of amide, alcohol, ester, or any combination thereof, and then adding an anti-solvent to precipitate a the crystal form B of compound of formula I. The anti-solvent referred to herein is a solvent in which compound of formula I has a lower solubility, selected from alkane or water. Furthermore, the introduction of the solvent can reduce the solubility of compound of formula I in the solution, thereby precipitating a solid crystal form B of compound of formula I.

Further preferably, when preparing a crystal form B of compound of formula I using a crystallization method, it comprises the following steps: mixing water with mixture X to obtain mixed solution A, followed by crystallization; wherein the mixture X comprises compound of formula I and N, N-dimethylacetamide (DMF); the volume-to-mass ratio of DMF to compound of formula I is 8 mL/g, and the volume-to-mass ratio of water to compound of formula I is 3 mL/g. The crystallization time can be 0.1-80 hours, for example, 1 hour. The crystallization temperature can be -15-60°C, for example, 5°C.

Further preferably, when preparing a crystal form B of compound of formula I using a crystallization method, it also comprises the following steps: mixing the mixed solution A with water, the volume-to-mass ratio of the water to compound of formula I is 5 mL/g, followed by crystallization. The crystallization time can be 0.5-72 h, for example, 0.5 h. The crystallization temperature can be 0-25°C, for example, 5°C.

Further preferably, when preparing a crystal form B of compound of formula I using a crystallization method, it also comprises the following post-processing: filtration and vacuum drying. The vacuum drying temperature can be 10-80°C, for example, 60°C.

The invention provides a method for preparing a stable single crystal form B of free-state PLX5622, aiming to be applicable for industrial production. The crystal form B prepared by this method exhibits significantly better pharmacological effects compared to the crystal form A. In comparison to crystal form A of compound of formula I, the crystal form B demonstrates outstanding efficacy in clearing microglia cells, with a higher rate of microglia cell clearance at 7 days or 14 days.

Another embodiment relates to a free-state PLX5622 (compound of formula I) crystal form C. The crystal form C of compound of formula I has an X-ray powder diffraction pattern comprising characteristic absorption peaks at diffraction angle 2Θ values of 8.24±0.2°, 13.16±0.2° and 16.40±0.2° in using Cu-Ku radiation.

Another object of the invention is to provide a crystal form C of compound of formula I, wherein the crystal form is a hydrate form.

The invention provides a crystal form C of compound of formula I, which has an X-ray powder diffraction pattern comprising characteristic absorption peaks at diffration angle 2Θ values of 8.24±0.2°, 13.16±0.2°, 16.40±0.2°, 18.38±0.2° and 27.66±0.2°.

The invention provides a crystal form C of compound of formula I, which has an X-ray powder diffraction pattern comprising characteristic absorption peaks at diffration angle 2θ values of 8.24±0.2°, 13.16±0.2°, 13.74±0.2°, 16.40±0.2°, 17.72±0.2°, 18.38±0.2°, 20.42±0.2° and 27.66±0.2°.

Preferably, the invention provides a crystal form C of compound of formula I, which has an X-ray powder diffraction pattern comprising characteristic absorption peaks at diffration angle 2Θ values of 8.24±0.2°, 9.26±0.2°, 13.16±0.2°, 13.74±0.2°, 16.40±0.2°, 17.72±0.2°, 18.38±0.2°, 20.42±0.2°, 24.72±0.2°, 27.24±0.2° and 27.66±0.2°.

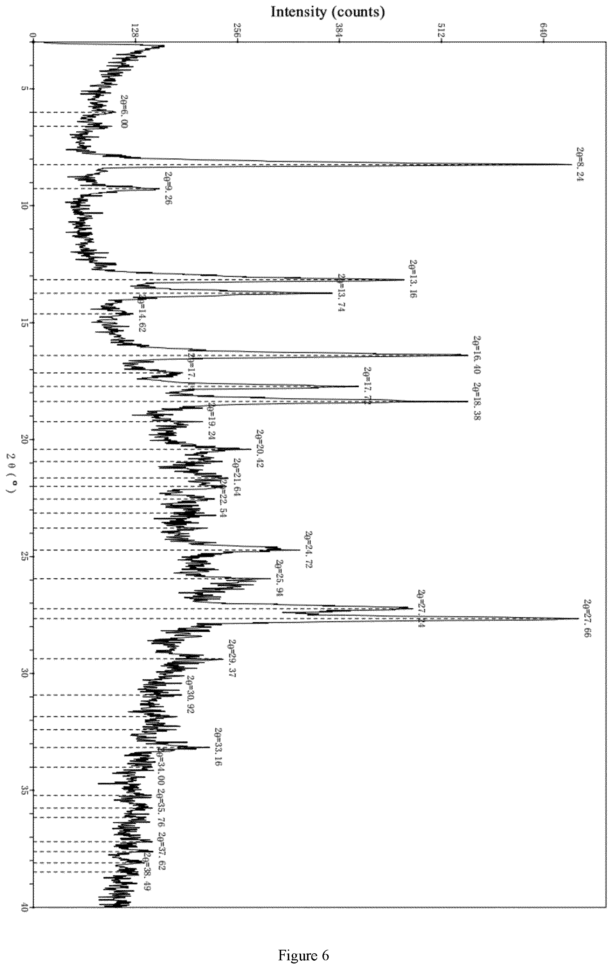

Further preferably, the crystal form C of compound of formula I has an XRPD pattern as shown in Figure 6.

The embodiments of the invention also provide a method for preparing Form C.

The embodiments of the invention also provide a method for preparing a crystal form C of compound of formula I, wherein compound I is obtained by crystallization in a mixed solvent of N, N-dimethylacetamide (DMAC) and water; preferably, the mixed solvent contains N, N-dimethylacetamide (DMAC) in a volume amount (mL) of 14 times and water in a volume amount (mL) of 7.5 times the mass (g) of compound of formula I.

Another embodiment relates to a free-state PLX5622 (compound of formula I) crystal form D. The crystal form D of compound of formula I has an X-ray powder diffraction pattern comprising characteristic absorption peaks at diffraction angle 2θ values of 11.70±0.2°, 17.76±0.2° and 15.92±0.2°using Cu-Kα radiation.

Another object of the invention is to provide a crystal form D of compound of formula I, wherein the crystal form is a solvate with one molecule of N, N-dimethylacetamide (DMAC).

The invention provides a crystal form D of compound of formula I, which has an X-ray powder diffraction pattern comprisingcharacteristic absorption peaks at diffraction angle 2θ values of 11.70±0.2°, 17.76±0.2°, 15.92±0.2°, 20.46±0.2° and 29.08±0.2°.

The invention provides a crystal form D of compound of formula I, which has an X-ray powder diffraction pattern comprising characteristic absorption peaks at diffraction angle 2θ values of 8.00±0.2°, 10.26±0.2°, 11.70±0.2°, 12.50±0.2°, 15.92±0.2°, 17.76±0.2°, 20.46±0.2° and 29.08±0.2°.

Preferably, the invention provides a crystal form D of compound of formula I, which has an X-ray powder diffraction pattern comprising absorption peaks at diffraction angle 2Θ values of 8.00±0.2°, 10.26±0.2°, 11.70±0.2°, 12.50±0.2°, 15.92±0.2°, 17.76±0.2°, 20.46±0.2°, 26.88±0.2°, 27.08±0.2°, 27.34±0.2°, 29.08±0.2° and 29.36±0.2°.

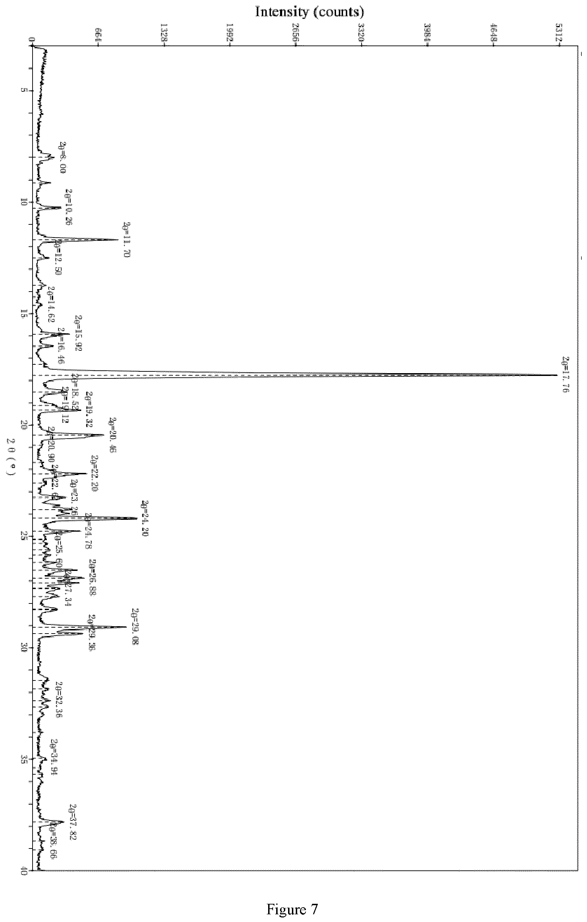

Further preferably, the crystal form D of compound of formula I has an XRPD pattern as shown in Figure 7.

Further preferably, the crystal form D of compound of formula I has a DSC curve with two endothermic peaks at 50°C-100°C and 189°C±3°C.

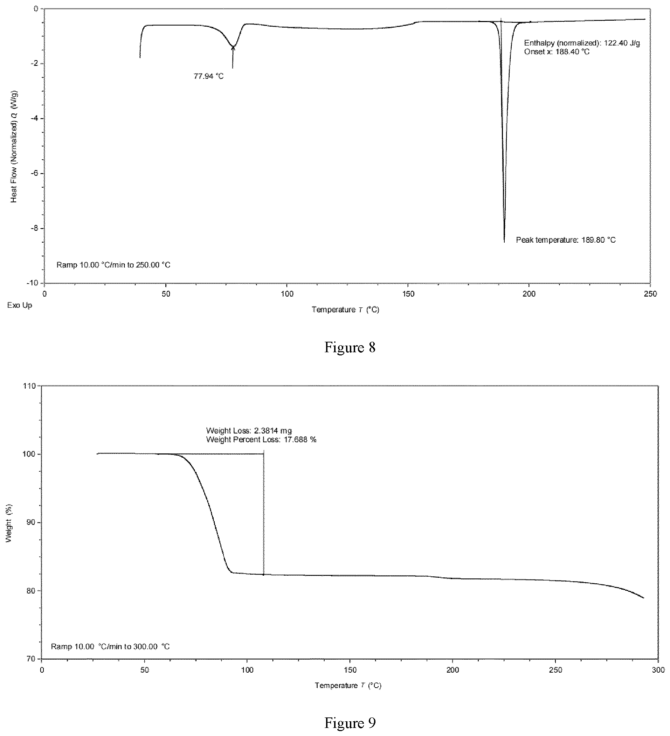

Further preferably, the crystal form D of compound of formula I has a DSC curve as shown in Figure 8.

Further preferably, the crystal form D of compound I has DSC curve substantially as shown in Figure 8, wherein the crystal form D exhibits two sharp endothermic peaks between 20°C-250°C, one of which is near 50°C-100°C, and the other is in the range of 189°C±3°C.

Further preferably, the DSC analysis method of crystal form D of compound of formula I as disclosed in the invention is as follows: the scanning rate is 10°C/min.

Further preferably, the crystal form D of compound of formula I has a TGA curve with a weight loss of 18% between 50-100°C.

Further preferably, the crystal form D of compound of formula I has a TGA spectrum of curve substantially as shown in Figure 9.

Further preferably, the crystal form D of compound of formula I has a TGA curve as shown in Figure 9, wherein the crystal form D exhibits an obvious weight loss at 50-100°C, and the weight loss mass is around 18%, corresponding to a molecule of N, N-dimethylacetamide (DMAC). Base on nuclear magnetic resonance, it is determined that the crystal form D is a solvate with one molecule of N, N-dimethylacetamide (DMAC).

Further preferably, the TGA analysis method of crystal form D of compound of formula I as disclosed in the invention is as follows: the scanning rate is 10°C/min.

The embodiments of the invention further provides a method for preparing crystal form D of compound of formula I, wherein the crystal form D of compound of formula I is obtained by crystallization in a mixed solvent of N, N-dimethylacetamide (DMAC) and water; Preferably, the mixed solvent contains N, N-dimethylacetamide (DMAC) in volume amount (mL) of 4 to 7 times and water in volume amount (mL) of 1 to 2.5 times the mass (g) of compound of formula I.

Another embodiment relates to a free-state PLX5622 (compound of formula I) crystal form E. The crystal form E of compound of formula I has an X-ray powder diffraction pattern comprising characteristic peaks at diffraction angle 2Θ values of 15.40±0.2°, 18.02±0.2° and 19.68±0.2°using Cu-Ku radiation.

Another object of the invention is to provide a crystal form E of compound of formula I, wherein the crystal form is a solvate of one molecule of N, N-dimethylacetamide (DMAC).

The invention provides a crystal form E of compound of formula I, which has an X-ray powder diffraction pattern comprising characteristic absorption peaks at diffraction angle 2Θ values of 15.40±0.2°, 18.02±0.2°, 19.68±0.2°, 21.72±0.2° and 26.20±0.2°.

The invention provides a crystal form E of compound of formula I, which has an X-ray powder diffraction pattern comprising characteristic absorption peaks at diffraction angle 2θ values of 10.24±0.2°, 13.44±0.2°, 15.40±0.2°, 17.16±0.2°, 18.02±0.2°, 19.68 ±0.2°, 20.82±0.2°, 21.72±0.2° and 26.20±0.2°.

Preferably, the crystal form E of compound of formula I as disclosed in the invention, which has an X-ray powder diffraction pattern comprising characteristic absorption at diffraction angle 2θ values of 8.98±0.2°, 9.86±0.2°, 10.24±0.2°, 13.44±0.2°, 15.40±0.2°, 16.14± 0.2°, 17.16±0.2°, 18.02±0.2°, 19.68±0.2°, 20.24±0.2°, 20.82±0.2°, 21.72±0.2°, 22.82±0.2°, 24.36±0.2°, 25.54±0.2°, 26.20±0.2°, 27.32±0.2°, 28.10±0.2°, 30.62±0.2°, 31.38±0.2°, 32.14±0.2° and 38.06±0.2°.

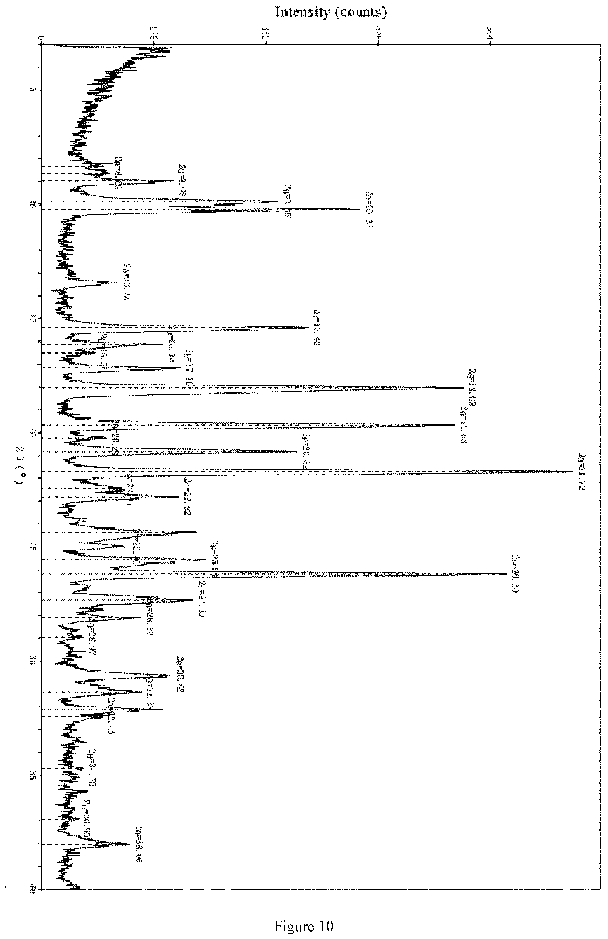

Further preferably, the crystal form E of compound of formula I has an XRPD pattern as shown in Figure 10.

Further preferably, the crystal form E of compound of formula I has a DSC curve with two endothermic peaks at 107 ± 3°C and 177 ± 3°C, and an exothermic peak at 111 ± 3°C.

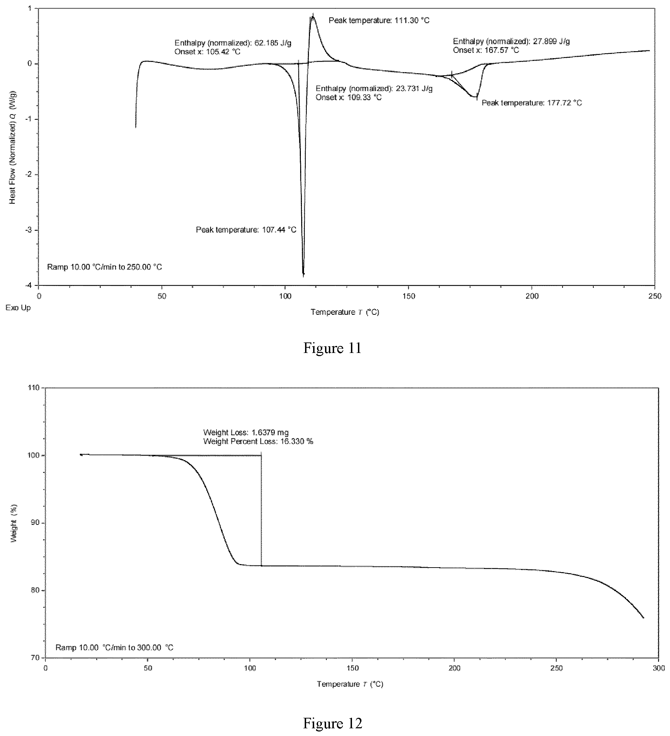

Further preferably, the crystal form E of compound of formula I has a DSC curve as shown in Figure 11.

Further preferably, the crystal form E of compound of formula I has a DSC curve substantially as shown in Figure 11, wherein the crystal form E exhibits multiple endothermic peaks between 20°C - 250°C. One relatively prominent endothermic peak is observed around 90°C - 120°C, while another is within the range of 150°C - 200°C.

Further preferably, the DSC analysis method of crystal form E of compound of formula I as disclosed in the invention is as follows: the scanning rate is 10°C/min.

Further preferably, the crystal form E of compound of formula I has a TGA curve with a weight loss of 16 % between 50 -110°C.

Further preferably, the crystal form E of compound of formula I has a TGA spectrum of curve as shown in Figure 12.

Further preferably, the crystal form E of formula I has a differential scanning calorimetry (TGA) curve as shown in Figure 12, wherein the crystal form E exhibits one significant weight loss of around 17% in the scanning temperature range of 20-300°C, indicating the crystalline is a solvate of one molecule of N, N-dimethylacetamide (DMAC).

Further preferably, the TGA analysis method of crystal form E of compound of formula I as disclosed in the invention is as follows: the scanning rate is 10°C/min.

The embodiments of the invention further provide a method for preparing a crystal form E of compound of formula I, wherein the compound of formula I is obtained by crystallization in a mixed solvent of N, N-dimethylacetamide (DMAC) and water. Preferably, at 25°C, the mixed solvent contains N, N-dimethylacetamide (DMAC) in a volume amount (mL) of 8 times and water in a volume amount (mL) of 5 times the mass (g) of compound of formula I. The mixture is then cooled to 5°C, incubated for 1-2 hours, followed by the addition of 7 times the volume amount (mL) of water relative to the mass (g) of compound of formula I, and further incubated for 0.5 hours. The mixture is then filtered to obtain the crystal form E.

Another embodiment relates to a free-state PLX5622 (compound of formula I) crystal form F. The crystal form F of compound of formula I has an X-ray powder diffraction pattern using comprising characteristic peaks at diffraction angle 2Θ values of 8.98±0.2°, 9.82±0.2° and 17.08±0.2°Cu-Kα radiation.

Another object of the invention is to provide a crystal form F of compound of formula I, wherein the crystal form is a solvate with THF.

The invention provides a crystal form F of compound of formula I, characterized by an X-ray powder diffraction pattern having peaks at diffraction angle 2Θ values of 8.98±0.2°, 9.82±0.2°, 17.08±0.2° and 19.72±0.2°.

The invention provides a crystal form F of compound of formula I, which has an X-ray powder diffraction pattern compring characteristic absorption peaks at diffraction angle 2θ values of 8.98±0.2°, 9.82±0.2°, 17.08±0.2°, 18.00±0.2°, 19.72±0.2°, 20.96±0.2°, 22.14±0.2° and 25.00±0.2°.

Preferably, the crystal form F of compound of formula I as disclosed in the invention, which has an X-ray powder diffraction pattern compring characteristic absorption peaks at diffraction angle 2θ values of 8.03±0.2°, 8.98±0.2°, 9.92±0.2°, 16.10±0.2°, 17.08±0.2°, 18.00± 0.2°, 19.72±0.2°, 20.96±0.2°, 22.14±0.2°, 24.26±0.2°, 25.00±0.2°, 27.18±0.2°, 28.60±0.2°, 29.75±0.2°, 34.54±0.2° and 36.50±0.2°.

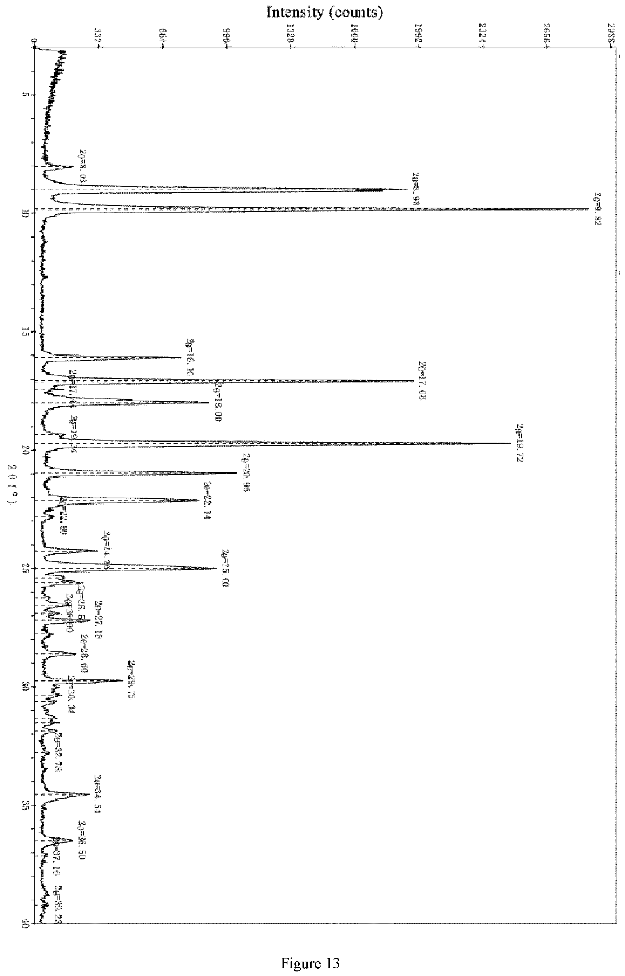

Further preferably, the crystal form F of compound of formula I has an XRPD pattern as shown in Figure 13.

Further preferably, the crystal form F of compound of formula I has a DSC curve with two endothermic peaks at 79±3°C and 190±3°C, and an exothermic peak at 182±3°C.

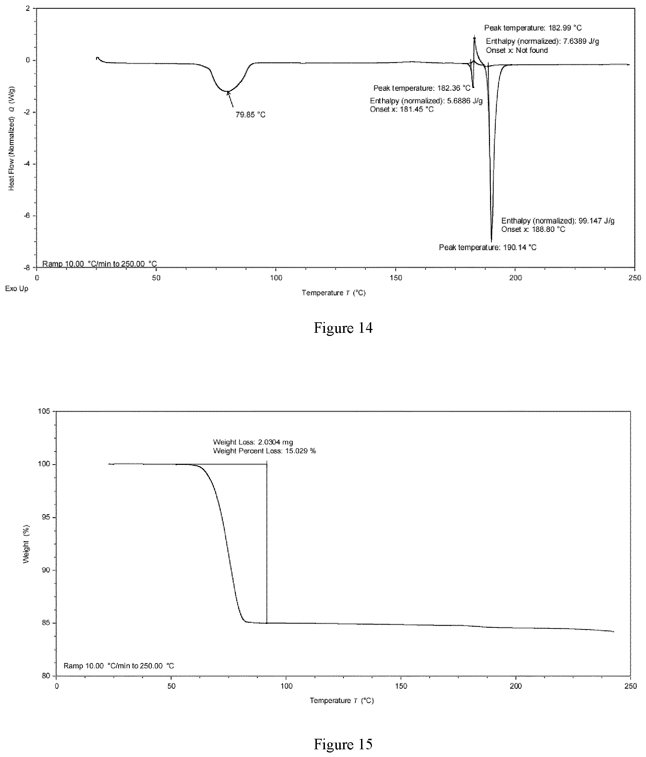

Further preferably, the crystal form F of compound of formula I has a DSC curve as shown in Figure 14.

Further preferably, the crystal form F of compound of formula I has a DSC curve substantially as shown in Figure 14, wherein the crystal form F exhibits multiple endothermic and exothermic events at 20°C-250°C. Among them, three relatively sharp endothermic peaks of which are seperately observed at around 78°C, the range of 182±3°C, and the range of 190±3°C.

Further preferably, the DSC analysis method of crystal form F of compound of formula I as disclosed in the invention is as follows: the scanning rate is 10°C/min.

Further preferably, the crystal form F of compound of formula I has a TGA curve of with a weight loss of 15 % at 50-100°C.

Further preferably, the crystal form F of compound of formula I has a TGA curve substantially as shown in Figure 15.

Further preferably, the crystal form F of compound of formula I has a TGA spectrum as shown in Figure 15, wherein the crystal form exhibits an obvious weight loss at 20-300°C, and the weight loss is about 15%, indicating the crystal form F of compound of formula I is a solvate with THF.

The embodiments of the invention also provide a method for preparing a crystal form F of compound of formula I, preferably by crystallizing compound of formula I from a solution of tetrahydrofuran. The volume-to-mass ratio of tetrahydrofuran solvent to compound of formula I can be 6-100 mL/g, for example, 7 mL/g.

In another embodiment of the invention, a pharmaceutical composition is provided, which comprising the group of compounds selected from crystal forms A, B, C, D, E and F, and pharmaceutically acceptable excipient.

Another embodiment relates to a method for treating subjects at risk of or suffering from glioblastoma, or another embodiment relates to a method for treating subjects with Alzheimer's disease (AD), or for restoring the function of microglial cells to ameliorate rare and common neurodegenerative diseases. The methods involve administering to the subject a therapeutically effective amount of crystal forms A, B, C, D, E and F, or the composition thereof.

In some embodiments, one or more solid, crystalline, or polymorphs of compound I as described herein, or a composition thereof as described herein, can be used to prepare pharmaceutical compositions for treating diseases or conditions selected from the group consisting of: for example, amnesia, Alzheimer's disease, cognitive impairment, motor and sensory dysfunction, improvement of cognitive impairment after TBI, binswager type dementia, dementia with lewy bodies, prosencephaly, microcephaly, cerebral palsy, addiction disorders, dependencies, tremors, Wilson's disease, vascular dementia, multi infarct dementia, frontotemporal dementia, pseudo-dementia, alopecia, baldness, wound healing, androgenetic alopecia (AGA), epilepsy, traumatic brain injury, hairy cell leukemia, non-small cell lung cancer, cleroderma, anterior eye disease, posterior eye disease, primary progressive multiple sclerosis, complex regional pain syndrome, viral infections (such as HIV infection), reflex sympathetic dystrophy, muscular dystrophy, causalgia, neuro-inflammation, neuroinflammatory disorders, bladder cancer, ureter cancer, urethra cancer, urachus cancer, basal cell carcinoma, cholangiocarcinoma, colon cancer, endometrial cancer, esophageal cancer, Ewing's sarcoma, gastric cancer, glioma, hepatocellular carcinoma, Hodgkin lymphoma, laryngeal carcinoma, leukemia, liver cancer, lung cancer, melanoma, mesothelioma, pancreatic cancer, rectal cancer, renal cancer, squamous cell carcinoma, t cell lymphoma, thyroid cancer, monocytic leukemia, pheochromocytoma, malignant peripheral nerve cell tumors, malignant peripheral nerve sheath tumors (MPNST), cutaneous and plexiform neurofibromas, leiomyoadenomatoid tumor, fibroids, uterine fibroids, leiomyosarcoma, papillary thyroid cancer, anaplastic thyroid cancer, medullary thyroid cancer, follicular thyroid cancer, hurthle cell carcinoma, thyroid cancer, angiosarcomas, liposarcomas, ascites, malignant ascites, mesothelioma, salivary gland tumors, mucoepidermoid carcinoma of the salivary gland, acinic cell carcinoma of the salivary gland, gastrointestinal stromal tumors, tumors that cause effusions in potential spaces of the body, pleural effusions, other sarcomas; tumor angiogenesis and paracrine tumor growth; and tumors that express aberrantly or otherwise CSFIR or activating mutations or translocations of any of the foregoing.

In another embodiment of the invention, the CSFIR mediated disease that can be treatable by any of crystal forms of compound of formula I in the invention is epilepsy.

In another embodiment of the invention, the CSFIR mediated disease that can be treatable by any of crystal forms of compound of formula I in the invention is traumatic brain injury.

In another embodiment of the invention, the CSFIR mediated disease that can be treatable by any of crystal forms of compound of formula I in the invention include tauopathies.

In another embodiment of the invention, the CSFIR mediated disease that can be treatable by any of crystal forms of compound of formula I in the invention include reducing viral reservoirs in patients.

In another embodiment of the invention, the CSFIR mediated disease that can be treatable by any of crystal forms of compound of formula I in the invention is anterior eye disease or posterior eye disease. Examples of these eye diseases include diseases of the cornea, conjunctiva, sclera, and lacrimal glands.

In another embodiment of the invention, the CSFIR mediated disease that can be treatable by any of crystal forms of compound of formula I in the invention include Erdheim Chester Disease/Langerhans cell histocytosis, hairy cell leukemia, and non-small cell lung cancer (NSCLC).

In another embodiment of the invention, disease that can be treatable by any of crystal forms of the compound of formula I in the invention is scleroderma. In this embodiment, the crystal forms can be administered in an external formulation such as a gel, cream or spray as non-limiting examples.

In one embodiment, the present disclosure provides a composition comprising the compound of this disclosure and a pharmaceutically acceptable carrier or excipient. The compound is selected from crystal forms A, B, C, D, E and F of compound of formula I.

In another embodiment, the invention provides a process of making a tablet or a capsule of containing crystal forms A, B, C, D, E and F of compound of formula I, comprising crystal forms A-F and a pharmaceutically acceptable carrier or excipient.

In another embodiment of the invention, a composition comprises the composition comprises crystal form A and form B of compound of formula I. In another embodiment, the composition comprises the crystal form A of compound of formula I and at least 10%, 15%, 20%, 25%, 30%, 35%, 40%, 45%, 50%, 55%, 60%, 65%, 70%, 75%, 80%, 85%, 90%, or 95% w/w of the crystal form B of compound of formula I. In yet another embodiment, the composition comprises the crystal form A and at least 50% of w/w of the crystal form B.

In another embodiment, the invention provides a process of making a tablet or a capsule containing the crystal form B of compound of formula I, comprising combining the crystal form B and a pharmaceutically acceptable carrier or excipient.

In another embodiment, the invention provides a process of making a tablet containing the crystal form B of compound of formula I, comprising combining the crystal form B and a pharmaceutically acceptable carrier or excipient.

In another embodiment, the composition comprises the crystal form B of compound of formula I. In another embodiment, the composition comprises at least 10%, 15%, 20%, 25%, 30%, 35%, 40%, 45%, 50%, 55%, 60%, 65%, 70%, 75%, 80%, 85%, 90%, or 95% w/w of the crystal form B.

In some embodiments, compositions used in the methods of the invention will comprise pharmaceutically acceptable carriers or excipients, such as fillers, binders, disintegrants, glidants, lubricants, solubilizers and surfactant. Examples of carriers include calcium carbonate, calcium phosphate, various sugars such as lactose, glucose or sucrose, types of starch, cellulose derivatives, gelatin, lipids, and the like.. Carriers also include physiologically compatible liquids as solvents or for suspensions, including, for example, sterile solutions of water for injection, saline solution, dextrose solutions, vegetable oils, mineral oils, animal oils, polyethylene glycols, liquid paraffin, and the like. Excipients may also include, for example, colloidal silica dioxide, silica gel, talc, magnesium silicate, calcium silicate, sodium aluminosilicate, microcrystalline cellulose, silicified microcrystalline cellulose, carboxymethyl cellulose, sodium dicarmellose, sodium benzoate, calcium carbonate, magnesium carbonate, stearic acid, aluminum stearate, calcium stearate, magnesium stearate, zinc stearate, magnesium oxide, starch, sodium starch glycolate , glyceryl monostearate, glyceryl behenate , glyceryl palmitostearate, hydrogenated vegetable oil, hydrogenated cotton seed oil, castor oil, mineral oil, polyethylene glycol (e.g. PEG 4000-8000), poloxamers, povidone , crospovidone , alginic acid, sodium docusate, cyclodextrin, polysorbates (e.g. polysorbate 80), magnesium lauryl sulfate, sodium lauryl sulfate, mannitol, xylitol, sorbitol, maltose, lactose, sucrose, fructose, calcium phosphate, dicalcium phosphate, calcium sulfate, dextran, dextrin, dextrose, cellulose acetate, maltodextrin, simethicone, polydextrosem, polydextrosem, gelatin, HPMC (hydroxypropyl methyl celluloses), HPC (hydroxypropyl cellulose), hydroxyethyl cellulose, and the like.

In some embodiments, oral administration can be used. Pharmaceutical preparations for oral use can be formulated into conventional oral dosage forms such as capsules, tablets, and liquid preparations such as syrups, elixirs, and concentrated drops. For example, tablets, coated tablets, hard capsules, soft capsules, solutions (e.g. aqueous, alcoholic, or oily solutions) and the like. Suitable excipients are, in particular, fillers such as sugars, including lactose, glucose, sucrose, mannitol, or sorbitol; cellulose preparations, for example, microcrystalline cellulose, corn starch, wheat starch, rice starch, potato starch, methyl cellulose, hydroxypropylmethyl-cellulose, gelatin, gum tragacanth, sodium carboxymethylcellulose (CMC), and/or polyvinylpyrrolidone (PVP: povidone); oily excipients, including vegetable and animal oils, such as sunflower oil, olive oil, or codliver oil. The oral dosage formulations may also contain disintegrating agents, such as the cross-linked polyvinylpyrrolidone, agar, or alginic acid, or a salt thereof such as sodium alginate; a lubricant, such as talc or magnesium stearate; a plasticizer, such as sorbitol; a sweetening such as sucrose, fructose, lactose, or aspartame; a natural or artificial flavoring agent, such as mint, strawberry, or cherry flavoring; or dye-stuffs or pigments, which can be used for identification or characterization of different doses or combinations. Also provided are dragee cores with suitable coatings. For this purpose, concentrated sugar solutions can be used, which may optionally contain, for example, gum arabic, talc, poly-vinylpyrrolidone, polyethylene glycol, and/or titanium dioxide, and suitable organic solvents or solvent mixtures.

Beneficial technical effects of the invention: the preparation method of the free-state PLX5622 solid forms of free-state PLX5622, i.e., new crystal forms A, B, C, D, E, and F of compound of formula I offer advantages such as easy operation, high yield, and suitability for industrial production; the obtained crystal forms have exhibit stable quality, high purity, and good solubility, making their discovery crucial for the subsequent development of formulations and pharmaceuticals. Researches show that the new crystal form A and crystal form B prepared possess favorable particle size distribution, stable physical and chemical properties during long-term storage, and good pharmacological effect. The inventors have observed that crystal form B demonstrates superior pharmacological effects compared to crystal form A; specifically, the crystal form B exhibits a notable efficacy in microglia cell clearance, with a better clearance rate of microglia cells at 7 or 14 days compared to crystal form A.

Based on current research results, crystal form A demonstrates relatively stable physicochemical properties and an acceptable pharmacological microglia elimination rate, although its efficacy is not as optimal as that of crystal form B, which offers enhanced stability and pharmacological efficacy. Hydrate crystal form C and solvate crystal forms D, E, and F, in their solid state, exhibit good crystallinity, particle size, and flowability. Their preparation methods are simple, and crystal forms A or B can be obtained after the removal of the solvent of crystal forms C-E, providing a new approach for the preparation of different target crystal forms.

### DESCRIPTION OF THE DRAWINGS

Figure 1 is an X-ray powder diffraction (XRPD) pattern of Form A of free-state PLX5622.
Figure 2 is an X-ray powder diffraction (XRPD) pattern of Form B of free-state PLX5622.
Figure 3 is differential scanning calorimetry (DSC) curve of Form B of free-state PLX5622.
Figure 4 is a polarizied light microscope (PLM) photo of Form B of free-state PLX5622 prepared by crystallization in Example 2.1.
Figure 5 is a polarized light microscope (PLM) photo of Form B of free-state PLX5622 prepared by spray drying in Example 2.2.
Figure 6 is an X-ray powder diffraction (XRPD) pattern of Form C of free-state PLX5622.
Figure 7 is an X-ray powder diffraction (XRPD) pattern of Form D of free-state PLX5622.
Figure 8 is a differential scanning calorimetry (DSC) curve of Form D of free-state PLX5622.
Figure 9 is a thermal gravimetric analysis (TGA) curve of Form D of free-state PLX5622.
Figure 10 is an X-ray powder diffraction (XRPD) pattern of Form E of free-state PLX5622.
Figure 11 is a differential scanning calorimetry (DSC) curve of Form E of free-state PLX5622.
Figure 12 is a thermal gravimetric analysis (TGA) curve of Form E of free-state PLX5622.
Figure 13 is an X-ray powder diffraction (XRPD) pattern of Form F of free-state PLX5622.
Figure 14 is a differential scanning calorimetry (DSC) curve of Form F of free-state PLX5622.
Figure 15 is a thermal gravimetric analysis (TGA) curve of Form F of free-state PLX5622.
Figure 16 is a diagram showing the effect of eliminating microglia after administration of From A and From B of free-state PLX5622 respectively.
Figure 17 is a the DSC-TGA curve of Form B of free-state PLX5622.
Figure 18 is a XRPD pattern of the mixture of From A and From B of free-state PLX5622.

### DETAILED DESCRIPTION OF THE DISCLOSURE

The following specific embodiments are provided to further illustrate the invention. It should be understood that these embodiments are only intended to illustrate the invention and not to limit the scope of the invention.

Experimental methods in the embodiments that are not specifically stated are typically conducted under conventional conditions or according to the conditions recommended by the manufacturer.

Raw materials or reagents used in the embodiments are commercially available unless otherwise specified.

Room temperature mentioned in the embodiments refers to 20-35°C. Unless otherwise indicated, the reagents mentioned are used without purification. All solvents are purchased from commercial suppliers, such as Aldrich, and used without further treatment.

The XRPD spectrum was collected on DX-21700BH diffractometer. The parameters of the X-ray powder diffraction method are as follows:
Radiation: Cu, Kα;
Tube voltage: 40 kilovolts (kV);
Tube current: 40 milliamps (mA);
Slits: 2# scattering slit: 1°, 3# anti-scattering slit: 1°, 4# receiving slit: 0.3mm;
Scan mode: step;
Step angle: 0.02°, sampling time: 1s;
Scan range: from 3.0 to 40.0 degrees;

The DSC curve was collected on TA Instruments Discovery DSC 250 instrument. The method parameters of the differential scanning calorimetry analysis are as follows:
Scan rate: 10°C/min;
Protective gas: nitrogen;

The TGA spectrum was collected on TA Instruments Discovery TGA 55 instrument. The method parameters of the thermal gravimetric analysis are as follows:
Scan rate: 10°C/min;
Protective gas: nitrogen;
Compound of formulate I, PLX5622, was prepared according to the route provided in Nature communications 2019; 10(1):3758.

### Example 1 Preparation of PLX5622 Form A

### Example 1.1

A mixture of 1g of compound of formula I and the excipient hydroxypropyl methylcellulose acetate succinate (HPMCAS) (mass ratio 1:1) was dissolved in 30mL of tetrahydrofuran (THF). The resulting solution was spray-dried at 80°C inlet temperature and mixed thoroughly to obtain a clear spray solution. The obtained solution was sprayed using a BUCHI Mini Spray Dryer B-290, with an inlet temperature of 95-100°C, outlet temperature of 80°C, and nitrogen flow rate of 450-605 L/hr. The resulting sample was vacuum-dried at 50±5°C for 40 hours to give an off-white solid, the X-ray powder diffraction pattern is shown in Figure.

### Example 12

A mixture of 10g of compound of formula I and the excipient hydroxypropyl methylcellulose acetate succinate (HPMCAS) (mass ratio 1:1) was dissolved in 30mL of tetrahydrofuran (THF). The resulting solution was spray-dried at 80°C inlet temperature and mixed thoroughly to obtain a clear spray solution. The obtained solution was sprayed using a BUCHI Mini Spray Dryer B-290, with an inlet temperature of 95-100°C, outlet temperature of 80°C, and nitrogen flow rate of 450-605 L/hr. The resulting sample was vacuum-dried at 50±5°C for 40 hours to give an off-white solid. The off-white solid was identified as From A of PLX5622 disclosed above.

### Example 1.3

A mixture of 100g of compound of formula I and the excipient hydroxypropyl methylcellulose acetate succinate (HPMCAS)(mass ratio 1:1) was dissolved in 30mL of tetrahydrofuran (THF). The resulting solution was spray-dried at 80°C inlet temperature and mixed thoroughly to obtain a clear spray solution. The obtained solution was sprayed using a BUCHI Mini Spray Dryer B-290, with an inlet temperature of 95-100°C, outlet temperature of 80°C, and nitrogen flow rate of 450-605 L/hr. The resulting sample was vacuum-dried at 50±5°C for 40 hours to give an off-white solid. The off-white solid was identified as From A of PLX5622 disclosed above. The melting point of Form A is 188-189 °C.

### Example 2: Preparation of PLX5622 Form B

### Example 2.1 Preparation of Form B By Crystallization Method

5g of the compound of formula I was dissolved in 40 mL (8v) N, N-dimethylacetamide (DMAC) at 5°C, followed by the addition of 15mL (3v) of water with stirring. The mixture was kept warm for 1 hour for crystallization, then 25mL (5v) of water was added, and the mixture was kept warm for another 0.5 hour. After filtration, the residue was vacuum dried 60°C to give Form B. The X-ray powder diffraction (XRPD) pattern is shown in Figure 2; differential scanning calorimetry (DSC) analysis is shown in Figure 3; polarized light microscope (PLM) photo is shown in Figure 4. The XRPD pattern of Form B showed no characteristic peaks at 8.60° and 10.50°, which are characteristic diffraction peaks of Form A, indicating that Form B does not contain Form A.

### Example 2.2 Preparation of Crystal form B by Solvent Removal Method

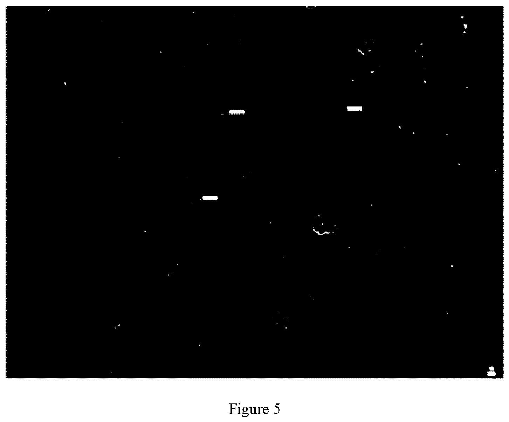

100g compound of formula I was dissolved in 3000mL THF, the mixture was stirred thoroughly to obtain a clear solution, which was sprayed using a BUCHI Mini Spray Dryer B-290, with an inlet temperature of 50-100°C and nitrogen flow rate of 450-605L/hr. The resulting sample was vacuum-dried at 25-60°C for 40 hours to give an off-white solid. The off-white solid was identified as From B of PLX5622 disclosed above. The polarizied light microscope (PLM) photo is shown in Figure 5.

### Example 3: Preparation of Form C

5g of the compound of formula I was dissolved in 60 mL (14v) N, N-dimethylacetamide (DMAC) at 25°C, followed by the addition of 37.5mL (7.5v) of water with stirring. The mixture was kept warm for 1 hour. After filtration, the residue was vacuum dried 60°C to give Form C. The XRPD pattern of the obtained Form C is shown in Figure 6.

### Example 4: Preparation of Form D

### Example 4.1

30g of the compound of formula I was dissolved in 210 mL (7v) N, N-dimethylacetamide (DMAC) at 25°C, followed by the addition of 75mL (2.5v) of water with stirring. The mixture was kept warm for a while, followed with filtration. The obtained example was an off-white solid. The XRPD pattern is shown in Figure 7, the differential scanning calorimetry (DSC) curve is shown in Figure 8; the thermal gravimetric analysis (TGA) curve is shown in Figure 9.

### Example 4.2

30g of the compound of formula I was dissolved in 120mL (4v) N, N-dimethylacetamide (DMAC) at 5°C, followed by the addition of 30mL (1v) of water with stirring. To the mixture was added crystal seed of From B, kept warm for a while and filtered. The obtained example was an off-white solid. The off-white solid was identified as From D of PLX5622 disclosed above.

### Example 5: Preparation of Form E

5g of the compound of formula I was dissolved in 40 mL (8v) N, N-dimethylacetamide (DMAC) at 25°C, followed by the addition of 5mL (1v) of water with stirring. The temperature was lowered to 5°C, and the mixture was incubated for 1-2 hours, and then 35mL (7v) of water was slowly added, and the mixture was further incubated for 0.5 hours, followed with filtration. The obtained example was an off-white solid. The XRPD pattern is shown in Figure 10, while the differential scanning calorimetry (DSC) analysis is shown in Figure 11. The thermal gravimetric analysis (TGA) curve is shown in Figure 12.

### Example 6: Preparation of Form F

5g of the compound of formula I was dissolved in 35mL (7v) tetrahydrofuran at room temperature. The temperature was lowered to 5°C, and the mixture was incubated for 1-2 hours, followed with filtration. The obtained sample was an off-white solid. The XRPD pattern is shown in Figure 14. The thermal gravimetric analysis (TGA) curve is shown in Figure 15.

### Example7: Drying transformation relationship and stability test of Form A, Form B, Form C, Form D, Form E and Form F.

Form A can remain stable when dried at high temperatures;
Form B can remain stable when dried at high temperatures;
Form C is a hydrate crystal form; this crystal form can only be obtained in a mixed system with high water content or a system in which only water. This form can be converted to Form A after high-temperature drying to remove residual solvent and water.

Form D is a solvate with one molecule of DMAC. After drying, the solvent is removed, converting it to Form A.

Form E is a solvate with one molecule of DMAC. After drying, it can be converted to Forms A and B to give a mixed crystal form.

Form F is a solvate with one molecule of THF. After drying, it can be converted to Forms A and B to give a mixed crystal form.

**Table 1 Summary of drying conditions for Form A-F**

| Starting crystal form | Drying conditions | Description | XRPD results |
|---|---|---|---|
| Form A | 60-80°C vacuum drying, 2 days | White to off-white powder | Form A |
| Form B | 60-80°C vacuum drying, 3 days | White to off-white powder | Form B |
| Form C | 60-80°C vacuum drying, 24 hours | White to off-white powder | Form A |
| Form D | 60-80°C vacuum drying, 24 hours | White to off-white powder | Form A |
| Form E | 60-80°C vacuum drying, 24 hours | White to off-white powder | Form A+B |
| Form F | 60-80°C vacuum drying, 24 hours | White to off-white powder | Form A+B |

### Stability Tests of Crystal form A of PLX5622 Compound of Formula I

**Table 2 Stability data of Form A**

| Storage conditions | Inspection time | XRPD | Purity (by HPLC%) | Individual Impurity (RRT/%) | |
|---|---|---|---|---|---|
| | | | | RRT=0.33 | RRT=1.50 |
| Initial | ODay | Form A | 99.90% | 0.06% | 0.04% |
| 40°C/75%RH | 5Days | Form A | 99.90% | 0.06% | 0.04% |
| 40°C/75%RH | 10Days | Form A | 99.90% | 0.06% | 0.04% |
| -20°C | 10 months | Form A | 99.90% | 0.06% | 0.04% |

The sampling analysis (XRPD) of the above samples during the test processing indicating that the PLX5622 Form A disclosed in the invention did not undergo any polymorphic changes under accelerated test conditions (40°C, 75% RH) and low-temperature storage conditions (-20°C), maintaining good physical and chemical stability.

### Stability Tests of Crystal form B of PLX5622 Compound of Formula I

**Table 3 Stability data of Form B**

| Storage conditions | Inspection time | XRPD | Purity (by HPLC%) | Individual Impurity (RRT/%) | |
|---|---|---|---|---|---|
| | | | | RRT=1.04 | RRT=1.29 |
| Initial | ODay | Form B | 99.92% | 0.05% | 0.03% |
| 40°C/75%RH | 5Days | Form B | 99.92% | 0.05% | 0.03% |
| 40°C/75%RH | 10Days | Form B | 99.92% | 0.05% | 0.03% |
| -20°C | 10 months | Form B | 99.92% | 0.05% | 0.03% |

Comparison under the same conditions found that the stability of PLX5622 Form B did not undergo any polymorphic changes under accelerated test conditions (40°C, 75% RH) and low-temperature storage conditions (-20°C), maintaining good physical and chemical stability as well.

### Example 8: Pharmacological Animal Experiment

1) Feeding: Mice were adaptively fed for 3-6 days upon arrival in the mouse room, with AIN-76A food. Group A was administered PLX5622 compound Form A, and Group B was administered PLX5622 compound Form B.
   Formal administration: Administration was conducted every other day, on days 1, 3, 5, 7, 9, 11, and 13. Each time of administration required weighing, and the amount was adjusted to 4 g per mouse (actual consumption around 3 g per mouse).
2) Sample collection: After anesthesia, mice were immobilized using tape or needles/pins, and the fur on the lateral thorax was lifted with forceps. The skin was then cut with scissors to expose the white sternum. The diaphragm was carefully incised with fine scissors, taking care not to damage the above heart.
   A small incision was made on the right atrium. The heart was fixed with forceps, and an injection needle was inserted into the left ventricle from the apex of the heart at an angle roughly parallel to the left-right midline of the heart. Deep red blood was observed to flow out, firstly perfused with saline, and followed by 4% paraformaldehyde fixation solution.
3) Sample processing:
   Sucrose gradient dehydration: The mouse brains were fixed in 4% paraformaldehyde overnight and then dehydrated with 20% sucrose solution until the material sank to the bottom, followed by dehydration with 30% sucrose solution until the material sank to the bottom. Embedding and sectioning: get 25-40µm sections, which are the tissues that need to be stained.
4) Staining
   1. After washing the sections, blocking was initiated with 5% BSA in PBST for 2 hours.
   2. Incubation with the primary antibody, IBA1 (1:500) in 1% BSA in PBST, at 4°C for at least 14 hours.
   3. Incubation with Alexa Fluor-conjugated secondary antibody (1:500) in 1% BSA in PBST, at room temperature for 2 hours.
   4. Washed with PBS 3-5 times for 10 minutes each, stained with DAPI, followed by addition of anti-fade reagent, dried overnight, and then photographed and scanned for analysis.
5) Statistics

Image j counting: All microglia with complete morphology need to be counted.

**Table 4 PLX5622-experimental result data of different groups**

| Group | Mouse breed/age | Solid form for administration | Number of mice | 7-day clearance rate/number of cells | 14-day clearance rate (number of cells) |
|---|---|---|---|---|---|
| Group A | C57BL/6J 10-11 weeks | Form A | 6 | 72.6% (1337) | 75.1% (922) |
| Group B | C57BL/6J 10-11 weeks | Form B | 7 | 87% (482) | 90% (377) |

Among them, the mice are all 3-month-old male mice; the clearance rates are the average clearance rate and the average statistical number of microglia (total number in brains).

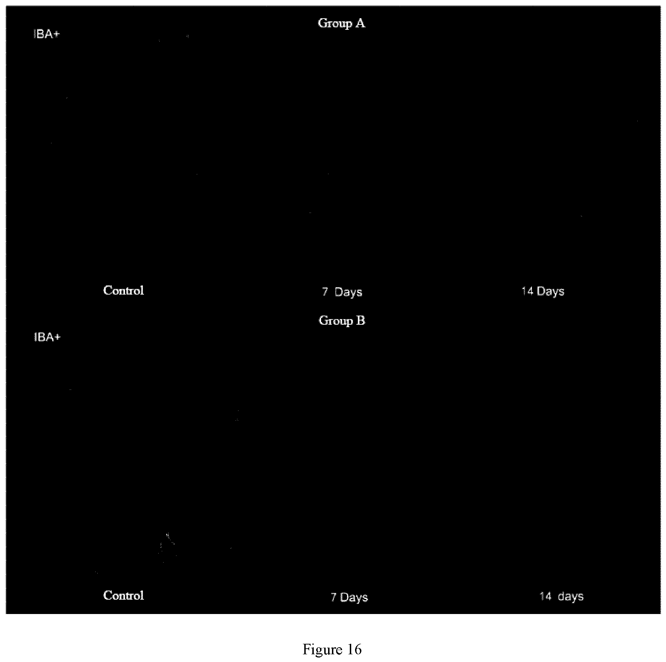

The test results in Table 4 indicate that the feeding clearance rate of group B is better, that is, Form B has a prominent effect in clearing microglia, and the 7-day clearance rate or 14-day clearance rate of microglia is better than Form A; the effect of eliminating microglial cells after administration of free-state PLX5622 Form A and Form B respectively is shown in Figure 16.

### Example 9: DSC-TGA detection of Form B

The DSC-TGA curve of Form B is shown in Figure 17. The DSC characterization exhibits an endothermic-exothermic effect at 181°C and an endothermic effect at 188°C for From B. The TGA characterization exhibits that there is almost no weight loss for Form B before 188°C.Based on Figure 13, it is indicated that Form B undergoes a phase transition at 181°C when heated.

In addition, the XRPD pattern of the mixture of Form A and Form B were tested using the aforementioned XRPD method. The results are shown in Figure 18. According to the XRPD pattern of Form A, Form B, and the mixture of Form A and Form B in Figure 18, it can be seen that the mixture of Form A and Form B exhibits characteristic diffraction peaks of Form A at 8.60° and 10.50°, while there are no such characteristic diffraction peaks in the XRPD pattern of Form B, indicating that Form B does not contain Form A.

Therefore, the crystal form B prepared in this invention is a pure crystal form.

### Example 10: Pharmacokinetic determination in rats

The different crystal forms of PLX5622 were evenly mixed in AIN-76A standard feed at a dose of 1200 ppm, with 7 rats per group divided into 7-day and 14-day groups. Plasma or brain samples from the 2 groups were collected for pharmacokinetic (PK) parameter analysis. For the 7-day group, plasma samples were collected on day 1, day 3, and day 7; after the rats were euthanized on day 7, brain tissue samples were collected. For the 10-day groups, plasma samples were collected from rats on day 10 and 14; on day 14, the mice were euthanized, and brain tissue samples were collected.

For the processing of plasma samples, rats were anesthetized on days 1, 3, 7 and 10 and 14, and approximately 150 µL of blood was collected via cardiac puncture into anticoagulant centrifuge tubes containing K ₂ EDTA. Within 15 minutes of sampling, plasma was obtained by centrifugation at 2000 g for 5 minutes at 4°C and stored at below -60°C until analysis. For the processing of brain tissue samples, the rats were euthanized on day 7 and 14, and then the brain tissue was collected. The residual blood on the surface of the tissue was washed away with normal saline, dried with filter paper, weighed, and frozen in dry ice, then store in a refrigerator below -60°C until sample analysis. Pharmacokinetic (PK) parameters Cmax and AUC were calculated based on a non-compartmental model using WinNonlin 8.2 software. Drug concentrations in mouse plasma and brain tissue were determined.

Experimental results showed that the Form B feed group exhibited better pharmacokinetic performance.

The above are merely preferred specific embodiments of the invention, and the protection scope of the invention is not limited thereto. Any modifications, equivalents, or variations made by those skilled in the art within the technical scope disclosed by the invention, based on the technical solution and inventive concept of the invention, should be encompassed within the scope of protection of the invention.

## Claims

1. A solid crystal form of compound of formula I, the structural formula of formula I is as follows: 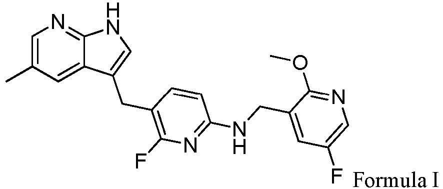
the solid crystal form of compound of formula I comprises crystal form A, crystal form B, crystal form C, crystal form D, crystal form E or crystal form F;
wherein the crystal form A has an X-ray powder diffraction pattern comprising characteristic absorption peaks at diffraction angle 2θ values of 8.60±0.2°, 10.50±0.2° and 13.80±0.2° using Cu-Kα radiation;
wherein the crystal form B has an X-ray powder diffraction pattern comprising characteristic peaks at diffraction angle 2θ values of 5.9±0.2°, 15.6±0.2° and 17.2±0.2° using Cu-Ku radiation; or, comprising characteristic peaks at diffraction angle 2θ values of 5.90±0.2°, 15.65±0.2° and 17.20±0.2°;
wherein the crystal form C has an X-ray powder diffraction pattern comprising characteristic peaks at diffraction angle 2θ values of 8.24±0.2°, 13.16±0.2° and 16.40±0.2° using Cu-Kα radiation;
wherein the crystal form D has an X-ray powder diffraction pattern comprising characteristic peaks at diffraction angle 2θ values of 11.70±0.2°, 17.76±0.2° and 15.92±0.2° using Cu-Kα radiation;
wherein the crystal form E has an X-ray powder diffraction pattern comprising characteristic peaks at diffraction angle 2θ values of 15.40±0.2°, 18.02±0.2° and 19.68±0.2° using Cu-Kα radiation;
wherein the crystal form F has an X-ray powder diffraction pattern comprising characteristic peaks at diffraction angle 2θ values of 8.98±0.2°, 9.82±0.2° and 17.08±0.2° using Cu-Kα radiation.

2. The solid crystal form according to claim 1, wherein the Form A has an X-ray powder diffraction pattern substantially as shown in Figure 1.

3. The solid crystal form of compound of formula I according to claim 1, wherein the solid crystal form of compound of formula I comprises one or more of the following conditions:
(1) wherein the crystal form A of compound of formula I has an X-ray powder diffraction pattern comprising characteristic peaks at 2θ values of 8.60±0.2°, 13.80±0.2°, 18.38±0.2°, and 20.90±0.2°; preferably, has an X-ray powder diffraction pattern comprising characteristic peaks at 2θ values of 8.60±0.2°, 10.50±0.2°, 13.80±0.2°, 14.66±0.2°, 17.18±0.2°, 18.38±0.2°, 20.90±0.2° and 25.98±0.2°; more preferably, has an X-ray powder diffraction pattern comprising characteristic peaks at 2θ values of 8.60±0.2°, 9.18±0.2°, 10.50±0.2°, 12.94±0.2°, 13.80±0.2°, 14.66±0.2°, 16.67±0.2°, 17.18±0.2°, 17.70±0.2°, 18.38±0.2°, 18.90±0.2°, 19.46±0.2°, 20.08±0.2°, 20.90±0.2°, 22.02±0.2°, 23.86±0.2°, 24.74±0.2°, 25.62±0.2°, 25.98±0.2°, 26.70±0.2°, 27.40±0.2°, 27.70±0.2°, 28.48±0.2°, 29.44±0.2°, 29.76±0.2°, 30.86±0.2° and 31.88±0.2°;
(2) wherein the crystal form B of compound of formula I has an X-ray powder diffraction pattern comprising characteristic peaks at 2θ values of 5.90±0.2°, 15.65±0.2°, 17.20±0.2° and 22.50±0.2°; preferably, has an X-ray powder diffraction pattern comprising characteristic peaks at 2θ values of 5.90±0.2°, 9.18±0.2°, 15.65±0.2°, 17.20±0.2°, 17.84±0.2°, 21.64±0.2°, 22.50±0.2° and 27.64±0.2°; more preferably, has an X-ray powder diffraction pattern comprising characteristic peaks at 2θ values of 5.90±0.2°, 9.18±0.2°, 11.70±0.2°, 14.16±0.2°, 15.65±0.2°, 17.20±0.2°, 17.84±0.2°, 19.86±0.2°, 21.64±0.2°, 21.99±0.2°, 22.50±0.2°, 23.88±0.2°, 24.28±0.2°, 24.52±0.2°, 27.64±0.2°, 28.10±0.2° and 28.40±0.2°;
(3) wherein the crystal form C of compound of formula I has an X-ray powder diffraction pattern comprising characteristic peaks at 2θ values of 8.24±0.2°, 13.16±0.2°, 16.40±0.2°, 18.38±0.2° and 27.66±0.2°; preferably, has an X-ray powder diffraction pattern comprising characteristic peaks at 2θ values of 8.24±0.2°, 13.16±0.2°, 13.74±0.2°, 16.40±0.2°, 17.72±0.2°, 18.38±0.2°, 20.42±0.2° and 27.66±0.2°; more preferably, has an X-ray powder diffraction pattern comprising characteristic peaks at 2θ values of 8.24±0.2°, 9.26±0.2°, 13.16±0.2°, 13.74±0.2°, 16.40±0.2°, 17.72±0.2°, 18.38±0.2°, 20.42±0.2°, 24.72±0.2°, 27.24±0.2° and 27.66±0.2°;
(4) wherein the crystal form D of compound of formula I has an X-ray powder diffraction pattern comprising characteristic peaks at 2θ values of 11.70±0.2°, 17.76±0.2°, 15.92±0.2°, 20.46±0.2° and 29.08±0.2°; preferably, has an X-ray powder diffraction pattern comprising characteristic peaks at 2Θ values of 8.00±0.2°, 10.26±0.2°, 11.70±0.2°, 12.50±0.2°, 15.92±0.2°, 17.76±0.2°, 20.46±0.2° and 29.08±0.2°; more preferably, has an X-ray powder diffraction pattern comprising characteristic peaks at 2θ values of 8.00±0.2°, 10.26±0.2°, 11.70±0.2°, 12.50±0.2°, 15.92±0.2°, 17.76±0.2°, 20.46±0.2°, 26.88±0.2°, 27.08±0.2°, 27.34±0.2°, 29.08±0.2° and 29.36±0.2°;
(5) wherein the crystal form E of compound of formula I has an X-ray powder diffraction pattern comprising characteristic peaks at 2Θ values of 15.40±0.2°, 18.02±0.2°, 19.68±0.2°, 21.72±0.2° and 26.20±0.2°; preferably, has an X-ray powder diffraction pattern comprising characteristic peaks at 2θ values of 10.24±0.2°, 13.44±0.2°, 15.40±0.2°, 17.16±0.2°, 18.02±0.2°, 19.68±0.2°, 20.82±0.2°, 21.72±0.2° and 26.20±0.2°; more preferably, has an X-ray powder diffraction pattern comprising characteristic peaks at 2θ values of 8.98±0.2°, 9.86±0.2°, 10.24±0.2°, 13.44±0.2° and 15.40±0.2°, 16.14±0.2°, 17.16±0.2°, 18.02±0.2°, 19.68±0.2°, 20.24±0.2°, 20.82±0.2°, 21.72±0.2°, 22.82±0.2°, 24.36±0.2°, 25.54±0.2°, 26.20±0.2°, 27.32±0.2°, 28.10±0.2°, 30.62±0.2°, 31.38±0.2°, 32.14±0.2° and 38.06±0.2°;
(6) wherein the crystal form F of compound of formula I has an X-ray powder diffraction pattern comprising characteristic peaks at 2Θ values of 8.98±0.2°, 9.82±0.2°, 17.08±0.2° and 19.72±0.2°; preferably, has an X-ray powder diffraction pattern comprising characteristic peaks at 2Θ values of 8.98±0.2°, 9.82±0.2°, 17.08±0.2°, 18.00±0.2°, 19.72±0.2°, 20.96±0.2°, 22.14±0.2° and 25.00±0.2°; more preferably, has an X-ray powder diffraction pattern comprising characteristic peaks at 2Θ values of 8.03±0.2°, 8.98±0.2°, 9.92±0.2°, 16.10±0.2°, 17.08±0.2°, 18.00±0.2°, 19.72±0.2°, 20.96±0.2°, 22.14±0.2°, 24.26±0.2°, 25.00±0.2°, 27.18±0.2°, 28.60±0.2°, 29.75±0.2°, 34.54±0.2° and 36.50±0.2°.

4. The solid crystal form of compound of formula I according to claim 1, wherein the solid crystal form of compound of formula I comprises one or more of the following conditions:
(1) wherein the crystal form B of compound of formula I has an XRPD pattern as shown in Figure 2;
(2) wherein the crystal form C of compound of formula I has an XRPD pattern as shown in Figure 6;
(3) wherein the crystal form D of compound of formula I has an XRPD pattern as shown in Figure 7;
(4) wherein the crystal form E of compound of formula I has an XRPD pattern as shown in Figure 10;
(5) wherein the crystal form F of compound of formula I has an XRPD pattern as shown in Figure 13.

5. The solid crystal form of compound of formula I according to claim 1, wherein the solid crystal form of compound of formula I comprises one or more of the following conditions:
(1) wherein the crystal form A of compound of formula I is an anhydrous crystal form;
(2) wherein the crystal form B of compound of formula I is an anhydrous crystal form;
(3) wherein the crystal form B of compound of formula I has a differential scanning calorimetry curve as shown in Figure 3 with endothermic peaks at 181°C±4°C and 188°C±4°C;
(4) wherein the crystal form D of compound of formula I has a differential scanning calorimetry curve as shown in Figure 8 with two relatively sharp endothermic peaks between 20°C-250°C, one of which is at 50°C-100°C, and the another is in the range of 189°C±3°C;
(5) wherein the crystal form D of compound of formula I has a differential scanning calorimetry curve as shown in Figure 9 with an obvious weight loss at 50-100°C and the weight loss mass percentage is 18%, the crystal form D of compound of formula I is a solvate with one molecule of N, N-dimethylacetamide;
(6) wherein the crystal form E of compound of formula I has a differential scanning calorimetry curve as shown in Figure 11 with multiple endothermic peaks in the range of 20°C-250°C; one relatively prominent endothermic peak of which is observed around 90°C-120°C, another is within the range of 150°C-200°C;
(7) wherein the crystal form E of compound of formula I has a differential scanning calorimetry curve as shown in Figure 12 with a significant weight loss of around 17% in the scanning temperature range of 20-300°C, wherein the crystal form E of compound of formula I is a solvate with one molecule of N, N-dimethylacetamide;
(8) wherein the crystal form F of compound of formula I has a differential scanning calorimetry curve as shown in Figure 14 with multiple endothermic and exothermic events at 20°C-250°C; three relatively sharp endothermic peaks of which are seperately observed at 78°C, 182°C±3°C, and 190°C±3°C;
(9) wherein the crystal form F of compound of formula I has a differential scanning calorimetry curve as shown in Figure 15 with an obvious weight loss at 20-300°C and the weight loss is about 15%, wherein the crystal form F of compound of formula I is a solvate with THF.

6. The solid crystal form of compound of formula I according to claim 1, wherein the solid crystal form of compound of formula I comprises one or more of the following conditions:
(1) wherein the crystal form A of compound of formula I has no solvent, and the solvent comprising water;
(2) wherein the crystal form B of compound of formula I has no solvent, and the solvent comprising water;
(3) wherein the crystal form B of compound of formula I further has an X-ray powder diffraction pattern comprising at least one or more characteristic peaks at 2θ values of 9.18±0.2°, 17.84±0.2°, 21.64±0.2°, 22.50±0.2° and 27.64±0.2°; more preferably, the X-ray powder diffraction pattern comprising at least one or more characteristic peaks at 2θ values of 11.70±0.2°, 14.16±0.2°, 19.86±0.2°, 21.99± 0.2°, 23.88±0.2°, 24.28±0.2°, 24.52±0.2°, 28.10±0.2° and 28.40±0.2°;
(4) wherein the crystal form B of compound of formula I has a differential scanning calorimetry curve with two endothermic peaks at 181°C±4°C and 188°C±4°C;
(5) wherein the crystal form D of compound of formula I has a DSC curve with two absorption peaks at 50°C-100°C and 189°C±3°C;
(6) wherein the crystal form D of compound of formula I has a differential scanning calorimetrycurve with a weight loss of 18% at 50-100°C;
(7) wherein the crystal form E of compound of formula I has a DSC curve with two endothermic peaks at 107±3°C and 177±3°C, and an exothermic peak at 111±3°C;
(8) wherein the crystal form E of compound of formula I has a TGA curve with a weight loss of 16 % at 50-110°C;
(9) wherein the crystal form E of compound of formula I has a DSC curve with two endothermic peaks at 79±3°C and 190±3°C, and an exothermic peak at 182±3°C;
(10) wherein the crystal form F of compound of formula I has a TGA curve with a weight loss of 15 % at 50-100°C.

7. The solid crystal form of compound of formula I according to claim 1, wherein the solid crystal form of compound of formula I comprises one or more of the following conditions:
(1) wherein the crystal form B of compound of formula I has a DSC curve substantially as shown in Figure 3;
(2) wherein the crystal form B of compound of formula I has a TGA curve substantially as shown in Figure 17;
(3) wherein the crystal form D of compound of formula I has a DSC curve substantially as shown in Figure 8;
(4) wherein the crystal form D of compound of formula I has a TGA curve substantially as shown in Figure 9;
(5) wherein the crystal form E of compound of formula I has a DSC curve substantially as shown in Figure 11;
(6) wherein the crystal form E of compound of formula I has a TGA curve substantially as shown in Figure 12;
(7) wherein the crystal form F of compound of formula I has a DSC curve substantially as shown in Figure 14;
(8) wherein the crystal form F of compound of formula I has a TGA curve substantially as shown in Figure 15.

8. A method for preparing a solid crystal form of compound of formula I according to claim 1, wherein the method is a method for preparing the crystal form B of compound of formula I, a method for preparing the crystal form C of compound of formula I, a method for preparing the crystal form D of compound of formula I, a method for preparing the crystal form E of compound of formula I or a method for preparing the crystal form F of compound of formula I;
wherein the method for preparing the crystal form B of compound of formula I is a solvent removal method or a crystallization method; preferably, the solvent removal method comprises the following steps: dissolving the compound of formula I in a tetrahydrofuran solution; spray drying to remove the solvent and obtaining the crystal form B of compound of formula I; further drying the crystal form B of compound of formula I; preferably, the crystallization method comprises the following steps: dissolving compound of formula I in a solvent of amide, alcohol, ester, or any combination thereof, then adding an anti-solvent to precipitate the crystal form B of compound of formula I; preferably, the amide solvent is N, N-dimethylacetamide, and preferably, the anti-solvent is an alkane or water;
wherein the method for preparing the crystal form C of compound of formula I comprises the following steps: crystallizing compound of formula I in a mixed solvent of N, N-dimethylacetamide DMAC and water to obtain the crystal form C of compound of formula I; preferably, the mixed solvent contains N, N-dimethylacetamide DMAC in a volume amount (mL) of 14 times and water in a volume amount (mL) of 7.5 times the mass (g) of compound of formula I;
wherein the method for preparing the crystal form D of compound of formula I comprises the following steps: crystallizing compound of formula I in a mixed solvent of N, N-dimethylacetamide DMAC and water to obtain the crystal form D of compound of formula I; preferably, the mixed solvent contains N, N-dimethylacetamide DMAC in a volume amount (mL) of 4-7 times and water in a volume amount of 1-2.5 times the mass (mL) of compound of formula I;
wherein the method for preparing the crystal form E of compound of formula I comprises the following step: crystallizing compound of formula I in a mixed solvent of N, N-dimethylacetamide DMAC and water to obtain the crystal form E of compound of formula I; preferably, the mixed solvent contains N, N-dimethylacetamide DMAC in a volume amount (mL) of 8 times and water in a volume amount (mL) of 5 times the mass (g) of compound of formula I; cooling the mixture to 5°C, incubating for 1-2 hours, adding 7 times the volume (mL) of water relative to the mass (g) of compound of formula I, further incubating for 0.5 hours, filtering and getting the crystal form E of compound of formula I;
wherein the method for preparing the crystal form E of compound of formula I comprises the following steps: crystallizing compound of formula I in a tetrahydrofuran to obtain Form F.

9. The method for preparing a solid crystal form of compound of formula I according to claim 8, wherein the method for preparing a solid crystal form of compound of formula I comprises one or more of the following conditions:
(1) wherein the volume-to-mass ratio of tetrahydrofuran to compound of formula I of the solvent removal method can be 15-100 mL/g, for example, 30 mL/g;
(2) wherein the crystallization method further includes the following steps: mixing water with mixture X to obtain mixed solution A, followed by crystallization; wherein the mixture X comprises the compound of formula I and N, N-dimethylacetamide; the volume-to-mass ratio of N, N-dimethylacetamide to compound of formula I is 8 mL/g, and the volume-to-mass ratio of water to compound of formula I is 3 mL/g; the crystallization temperature can be -15-60°C, for example, 5°C; preferably, the crystallization method comprises the following steps: mixing the mixed solution A with water, the volume-to-mass ratio of the water to compound of formula I is 5 mL/g, followed by crystallization; the crystallization temperature can be 0-25°C, for example, 5°C.

10. The solid crystal form of compound of formula I according to claim 1, wherein the solid crystal form of compound of formula I comprises any one of the following schemes:
Scheme 1, the crystal form B of compound of formula I comprises:
i) an X-ray powder diffraction pattern comprising characteristic peaks at 2Θ values of 5.90±0.2°, 9.18±0.2°, 11.70±0.2°, 14.16±0.2°, 15.65±0.2°, 17.20±0.2°, 17.84±0.2°, 19.86±0.2°, 21.64±0.2°, 21.99±0.2°, 22.50±0.2°, 23.88±0.2°, 24.28±0.2°, 24.52±0.2°, 27.64±0.2°, 28.10±0.2° and 28.40±0.2°;
ii) an X-ray powder diffraction pattern substantially as shown in Figure 2;
iii) a differential scanning calorimetry DSC curve with endothermic peaks near 181°C±4°C and 188°C±4°C;
iv) a DSC curve substantially as shown in Figure 3;
Scheme 2, the crystal form B of compound of formula I is prepared by the following method:
Method 1 solvent removal method:
i) dissolving compound of formula I in a solvent of ethers, alkanes, alcohols, esters, water or any combination thereof, then removing the solvent to obtain the compound of formula I; wherein the method of solvent removal is selected from distillation or spray drying;
ii) using ether solvent to dissolve, followed by spray drying;
iii) dissolving compound of formula I in tetrahydrofuran solution; spray drying to remove the solvent and obtaining the crystal form B of compound of formula I; optionally further drying the crystal form B of compound of formula I;
Method 2 crystallization method:
1) dissolving the compound of formula I in amides, alcohols, esters or any combination thereof, adding an anti-solvent to crystallize and precipitate the crystal form compound of formula I; the preferred amide is N, N-dimethylacetamide, and the preferred anti-solvent is an alkane or water;
Scheme 3, comprising:
i) an X-ray powder diffraction pattern comprising characteristic peaks at 2Θ values of 8.24±0.2°, 9.26±0.2°, 13.16±0.2°, 13.74±0.2°, 16.40±0.2°, 17.72±0.2°, 18.38±0.2°, 20.42±0.2°, 24.72±0.2°, 27.24±0.2° and 27.66±0.2°;
ii) an X-ray powder diffraction pattern substantially as shown in Figure 6;
iii) wherein the crystal form C of compound of formula I is prepared by comprising the following method:
crystallizing compound of formula I in a mixed solvent of N, N-dimethylacetamide DMAC and water to obtain the crystal form B of compound of formula I; preferably, the mixed solvent contains N, N-dimethylacetamide in a volume amount (mL) of 14 times and water in a volume amount (mL) of 7.5 times the mass (g) of compound of formula I;
Scheme 4, the crystal form D of compound of formula I comprises:
i) an X-ray powder diffraction pattern comprising characteristic peaks at 2θ values of 8.00±0.2°, 10.26±0.2°, 11.70±0.2°, 12.50±0.2°, 15.92±0.2°, 17.76±0.2°, 20.46±0.2°, 26.88±0.2°, 27.08±0.2°, 27.34±0.2°, 29.08±0.2° and 29.36±0.2°;
ii) an X-ray powder diffraction pattern substantially as shown in Figure 7;
iii) a differential scanning calorimetry DSC curve with two relatively sharp endothermic peaks at 20°C-250°C, one of which is near 50°C-100°C, the another is in the range of 189°C±3°C;
iv) a DSC curve substantially as shown in Figure 8;
v) comprising an obvious weight loss at 50-100°C and the weight loss mass percentage is about 18%; the crystal form D of compound of formula I is a solvate with one molecule of N, N-dimethylacetamide;
vi) a TGA curve substantially as shown in Figure 9;
vii) wherein the crystal form D of compound of formula I is prepared by comprising the following method:
crystallizing compound of formula I in a mixed solvent of N, N-dimethylacetamide and water to obtain the crystal form D of compound of formula I; preferably, the mixed solvent contains N, N-dimethylacetamide in a volume amount (mL) of 4 to 7 times and water in a volume amount (mL) of 1 to 2.5 times the mass (g) of compound of formula I;
Scheme 5, the crystal form E of compound of formula I comprises:
i) an X-ray powder diffraction pattern with characteristic peaks at 2Θ values 8.98±0.2°, 9.86±0.2°, 10.24±0.2°, 13.44±0.2°, 15.40±0.2°, 16.14±0.2°, 17.16±0.2°, 18.02±0.2°, 19.68±0.2°, 20.24±0.2°, 20.82±0.2°, 21.72±0.2°, 22.82±0.2°, 24.36±0.2°, 25.54±0.2°, 26.20±0.2°, 27.32±0.2°, 28.10±0.2°, 30.62±0.2°, 31.38±0.2°, 32.14±0.2° and 38.06±0.2°;
ii) an X-ray powder diffraction pattern substantially as shown in Figure 10;
iii) a differential scanning calorimetry DSC curve contains two relatively obvious and sharp endothermic peaks at 20°C-250°C; one of which is near 90°C-120°C, and the another is in the range of 150°C-200°C;
iv) a DSC curve substantially as shown in Figure 11;
v) comprising an obvious weight loss at 50-100°C and the weight loss mass percentage is about 17%; the crystal form E of compound of formula I is a solvate with one molecule of N, N-dimethylacetamide;
vi) a TGA curve substantially as shown in Figure 12;
vii)wherein the crystal form E of compound of formula I is prepared by comprising the following method:
crystallizing compound of formula I in a mixed solvent of N, N-dimethylacetamide and water to obtain the crystal form E of compound of formula I; preferably, the mixed solvent contains N, N-dimethylacetamide in a volume amount (mL) of 8 times and water in a volume amount of 5 times the mass (g) of compound of formula I; cooling the mixture to 5°C, incubating for 1-2 hours, adding water in a volume amount (mL) of 7 times the mass (g) of compound of formula I, and further incubated for 0.5 hours, filtered to obtain the crystal form E of compound of formula I;
Scheme 6, the crystal form F of compound of formula I comprises:
i) an X-ray powder diffraction pattern comprising characteristic peaks at 2Θ values of 8.03±0.2°, 8.98±0.2°, 9.92±0.2°, 16.10±0.2°, 17.08±0.2°, 18.00±0.2°, 19.72±0.2°, 20.96±0.2°, 22.14±0.2°, 24.26±0.2°, 25.00±0.2°, 27.18±0.2°, 28.60±0.2°, 29.75±0.2°, 34.54±0.2° and 36.50±0.2°;
ii) an X-ray powder diffraction pattern substantially as shown in Figure 13;
iii) a differential scanning calorimetry DSC curve with three relatively sharp endothermic peaks at 20°C-250°C, which are seperately observed at 78°C, 182°C±3°C, and 190°C±3°C;
iv) a DSC curve substantially as shown in Figure 14;
v) comprising an obvious weight loss at 20-300°C, and the weight loss mass percentage is about 15%; the crystal form F of compound of formula I is a solvate with THF;
vi) a TGA curve substantially as shown in Figure 15;
vii) wherein the crystal form F of compound of formula I is prepared by comprising the following methods:
crystallizing compound of formula I in tetrahydrofuran solution to obtain the crystal form F of compound of formula I.

11. Use of the solid crystal form of compound of formula I according to any one of claims 1-7 and 10 for preparing drugs to treat subjects with or at risk of neurofibromatosis or Alzheimer's disease, or preparing drugs for restoring the function of microglial cells, to improve subjects afflicted with rare and common neurodegenerative diseases.
